# EUROPÄISCHE PATENTSCHRIFT

(11) **EP 2 222 179 B1**
(45) Veröffentlichungstag und Bekanntmachung des Hinweises auf die Patenterteilung: **30.01.2013**
(21) Anmeldenummer: 08850563.1
(22) Anmeldetag: 14.11.2008
(51) Int. Cl.: A23D 9/007, A23D 9/02, A23L 1/221, A23D 9/00, C11B 1/06

(54) **VERFAHREN ZUR ÜBERTRAGUNG UND KONSERVIERUNG VON AROMA- UND INHALTSSTOFFEN VON PFLANZEN UND/ODER PFLANZENTEILEN AUF EIN ÖL, ANGEREICHERTES ÖL UND DESSEN VERWENDUNGEN**
METHOD FOR TRANSFERRING AND PRESERVING AROMATIC SUBSTANCES AND CONSTITUENTS OF PLANTS AND/OR PLANT PARTS TO AN OIL, ENRICHED OIL AND USES THEREOF
PROCÉDÉ POUR TRANSFÉRER ET CONSERVER DES AROMATISANTS ET CONSTITUANTS DE PLANTES ET/OU DE PARTIES DE PLANTES DANS UNE HUILE, HUILE ENRICHIE ET SES UTILISATIONS

(30) Priorität: 14.11.2007 DE 102007055007
(43) Veröffentlichungstag der Anmeldung: 01.09.2010
(73) Patentinhaber: Mueller, Baerbel, 97799 Zeitlofs (DE)
(72) Erfinder: Mueller, Baerbel, 97799 Zeitlofs (DE)
(74) Vertreter: Gulde Hengelhaupt Ziebig & Schneider
(86) Internationale Anmeldenummer: PCT/EP2008/065611
(87) Internationale Veröffentlichungsnummer: WO 2009/063077

(56) Entgegenhaltungen:
- GB-A- 1 237 042
- JP-A- 8 214 831
- US-A- 3 071 475
- US-B1- 6 497 908

## Beschreibung

Die Erfindung betrifft ein Verfahren zur Übertragung und Konservierung von Aroma- und Inhaltsstoffen von frischen, getrockneten und/oder verarbeiteten Früchten, Samen, Pflanzen und/oder Pflanzenteilen auf ein Öl, ein angereichertes Öl sowie dessen Verwendungen.

Olivenöl ist ein pflanzliches Öl, das insbesondere im Mittelmeerraum wesentlicher Bestandteil der Küche ist und dem diverse gesundheitsförderliche Eigenschaften zugeschrieben werden. Olivenöl besteht wie alle Pflanzenöle aus an Glycerin gebundenen Fettsäuren, von denen 77 % einfach ungesättigt, 9 % mehrfach ungesättigt und 14 % gesättigt sind. Darüber hinaus enthält Olivenöl eine Vielzahl von sekundären Pflanzenstoffen, die wesentlich für die besonderen Eigenschaften des Olivenöls sind.

So haben Studien gezeigt, dass der Verzehr von Olivenöl positive Auswirkungen auf die Blut-Cholesterinwerte hat und das Auftreten von koronaren Erkrankungen reduziert. Auch die Gefahr bestimmte Krebserkrankungen zu entwickeln, soll durch Olivenöl reduziert werden.

Es ist bekannt, dass Olivenöl eine konservierende Wirkung aufweist. Daher wird dieses zum Einlegen von beispielsweise getrockneten Tomaten, Knoblauch und dergleichen verwendet. Weiterhin ist es üblich, Olivenöl mit Kräutern, Zitronenauszügen, ätherischen Ölen oder künstlichen Aromen zu versetzen. Die entsprechend hergestellten Produkte sind jedoch in der Regel minderwertig. Werden beispielsweise getrocknete Gewürze in Olivenöl eingelegt, gehen nur sehr wenige Aromen auf das Öl über und der Prozess dauert Wochen oder Monate. Aromatisierte Öle sind beispielsweise in den Dohumenlen US 3071475, GB 1237042 und US 6497908 offenbart.

Es ist Aufgabe der Erfindung, ein Verfahren zur Übertragung und Konservierung von Aroma- und Inhaltsstoffen von frischen, getrockneten und/oder verarbeiteten Früchten, Samen, Pflanzen und/oder Pflanzenteilen auf ein Öl zu schaffen und damit ein mit Aroma- und Inhaltstoffen angereichertes Öl bereitzustellen, das für Verwendungen in den verschiedensten Bereichen geeignet ist.

Diese Aufgabe wird durch die Merkmale der Ansprüche 1, 11, 12-15 gelöst.

Erfindungsgemäß ist ein Verfahren zur Übertragung und Konservierung von Aroma- und Inhaltsstoffen von frischen, getrockneten und/oder verarbeiteten Früchten, Samen, Pflanzen und/oder Pflanzenteilen auf ein Öl vorgesehen, bei dem zumindest bei einem Verfahrensschritt während der Herstellung des Öls aus ölhaltigen Früchten, Samen, Pflanzen oder Pflanzenteilen, die einen, vorzugsweise hohen Anteil an Antioxidantien aufweisen, die aroma- und Inhaltsstoffgebenden Früchte, Samen, Pflanzen, Pflanzenteile, verarbeitete Pflanzen und/oder Pflanzenteile hinzugefügt und mit verarbeitet werden, anschließend das während des Verfahrens entstandene Öl-/Wassergemisch getrennt und ein angereichertes Öl erhalten wird.

Wesentlich bei dem erfindungsgemäßen Verfahren ist, dass die aroma- und Inhaftsstoffgebenden Bestandteile bei der Herstellung des Öls zu einem Zeitpunkt zugegeben werden, an dem noch keine Trennung des in den ölhaltigen Bestandteilen enthaltenen Fruchtwassers vom Öl erfolgt ist. Besonders bevorzugt findet die Zugabe der aroma- und Inhaltsstoffgebenden Bestandteile in Gegenwart der festen Bestandteile statt, also vor Abtrennung des Tresters.

Die wesentlichen Schritte bei der Ölherstellung sind
a) mechanische Zerkleinerung der ölhaltigen Bestandteile zu einem Brei,
b) Rühren des Breis,
c) Abtrennung des im Brei enthaltenen Öl-/Wassergemisch von den festen Bestandteilen und
d) Trennung von ÖI- und Wasserphase.

Wie bereits ausgeführt, erfolgt die Zugabe der aroma- und inhaltsstoffgebenden Bestandteile vorzugsweise bei Schritt a) und/oder b), wobei durch den Zerkleinerungsprozess und das Rühren eine besonders innige Durchmischung der Bestandteile erfolgt.

Die aroma- und inhaltsstoffgebenden Bestandteile können verarbeitet oder unverarbeitet zugefügt werden, so werden vorzugsweise Bananen direkt mit dem Brei verarbeitet, während Zitrusfrüchte separat zerkleinert und mit dem Brei gerührt werden.

Das gemeinsame Zerkleinern soll etwa über einen Zeitraum von 10-15 Minuten erfolgen. Eine längerer Zerkleinerungsvorgang würde zunehmend gewünschte Inhaltsstoffe zerstören.

Das Rühren erfolgt vorzugsweise unter Schutzgas für einen Zeitraum von 20 bis 45 Minuten miteinander gerührt werden, wobei je nach aroma- und inhaltsstoffgebenden Bestandteilen die genaue Zeitdauer bestimmt wird. Im Einzelfall kann es auch sinnvoll sein, den Brei ohne Rühren für eine längere Zeit, beispielsweise über Nacht stehen zu lassen.

Überaschenderweise erfolgt die stärkste Anreicherung des Öls bei der Verarbeitung des Breis. Dies lässt sich bei der Zugabe in anderen Verfahrensschritten nach Abtrennung der festen Bestandteile/Trester nicht erreichen, auch wenn dabei die Kontaktdauer der einzelnen Bestandteile verlängert wird.

Bei Zugabe der aroma- und Inhaltsstoffgebenden Bestandteile zum Öl-/Wassergemisch sind diese vorzugsweise bereits verarbeitet, beispielsweise zerkleinert. Dieses Gemisch wird für eine bestimmte Zeitdauer miteinander in Kontakt belassen und dabei vorzugsweise stetig durchmischt. Diese Zeitdauer entspricht im wesentlichen den o.g. Angaben zur Zeitdauer. Bei dieser Variante erfolgt in der Regel eine geringere Anreicherung und es müssen nochmals feste Bestandteile abgetrennt werden, jedoch kann es bei bestimmten Pflanzen bzw. Früchten vorteilhaft sein, die Übertragung der Inhaltsstoffe in diesem Schritt vorzunehmen. Bei Bedarf können auch beide Varianten miteinander kombiniert werden.

Das im Brei enthaltene Öl-/Wassergemisch wird vorzugsweise mittels eines Dekanters gewonnen.

Durch einen Dekanter kann auch die Trennung des Öl-/Wassergemischs erfolgen.

Mitunter wird vorher noch eine bestimmte Menge an Wasser beim Dekantieren zugegeben, um beispielsweise unerwünschte lnhaltstoffe, die den Geschmack prägen können, auszutragen.

Anschließend kann das erhaltene angereicherte Öl zentrifugiert und bei Bedarf filtriert weiterverarbeitet werden. Alternativ zur Filtration erfolgt eine Sedimentation im Lagerbehälter. Durch diese Schritte wird u.a. die Haltbarkeit des angereicherten Öls erhöht.

Auch alle anderen physikalischen Herstellungsverfahren, z. B. mittels einer Schneckenpresse, die eine Ölgewinnung unter gleichzeitiger Zugabe von frischen, getrockneten und/oder verarbeiteten Früchten, Samen, Pflanzen und/oder Pflanzenteilen erlauben, sind von der Erfindung mitumfasst.

Aufgrund des hohen Anteils an antioxidativen Substanzen aus dem Öl kann vorteilhafterweise auf die Zugabe von Zusätzen zur Konservierung verzichtet werden.

Wesentlich bei der Herstellung der angereicherten Öls ist, dass dieser Vorgang ohne Erwärmung erfolgt. Vorzugsweise liegt die Temperatur unter 28 °C, besonders bevorzugt unter 25 °C.

Das angereicherte Öl kann zusätzlich noch in herkömmlicher Weise aromatisiert werden, indem entsprechende Kräuter, Früchte oder dergleichen im Öl eingelegt und später wieder separiert werden.

Zur Herstellung des Öls werden ölhaltige Früchte, Samen, Pflanzen oder Pflanzenteile verwendet, die insbesondere einen hohen Anteil an Antioxidantien aufweisen, im Vergleich zu beispielsweise Ölsaaten.

Vorzugsweise werden für das erfindungsgemäße Verfahren Oliven eingesetzt, wobei Sorten mit einem hohen Antioxidantiengehalt besonders bevorzugt werden. Hierbei werden insbesondere Oliven der Sorte Coratina verwendet.

Auch andere ölhaltige Pflanzen können erfindungsgemäß eingesetzt werden, wenn diese insbesondere reich an Antioxidantien sind, wie beispielsweise die Früchte des Arganbaums, die sich insbesondere durch einen sehr hohen Gehalt an Tocopherolen auszeichnen, oder Traubenkerne.

Es wird bevorzugt, die Oliven innerhalb 48 Stunden nach der Ernte zu verarbeiten, um die für die Eigenschaften des erfindungsgemäß angereicherten Öls wesentlichen Inhaltsstoffe in größtmöglicher Menge zu erhalten.

Die Verarbeitung von unreifen, grünen Oliven wird besonders bevorzugt, da in diesem Zustand der Gehalt an Antioxidantien besonders hoch ist.

Die erfindungsgemäß zu verwendenden Oliven weisen Inhaltsstoffe auf, die antioxidativ wirken. Dazu zählen:
- Tocopherole, die üblicherweise zu 90% als α-Tocopherol vorliegen,
- Phenolische Verbindungen, einfache Phenole, Oleuropein, Flavonoide umfassend, die zudem noch antithrombotische Eigenschaften besitzen,
- Kohlenwasserstoffe, wie Squalen, das zudem protektiv gegenüber Krebserkrankungen wirkt.

Ferner enthält Olivenöl Sterole, die den Cholesterinspiegel im menschlichen Blut beeinflussen und auch gegenüber Krebserkrankungen protektiv wirken sollen sowie Inhaltsstoffe mit antimikrobiellen Eigenschaften.

Wesentlich für das erfindungsgemäße Verfahren ist ein möglichst hoher Gehalt an Antioxidantien. Dieser sollte für α-Tocopheral mindestens im Bereich von 24 bis 43 mg/100 g Olivenöl und für Squalen im Bereich von 400 bis 700 mag/100 g Olivenöl liegen. Für Polyphenole sollte der Gehalt vorzugsweise mindestens 100 mg/l, besonders bevorzugt mindestens 300 mg/l, am bevorzugtesten mindestens 500 mg/l betragen.

Auch ein hoher Gehalt an antimikrobiellen Verbindungen wird erfindungsgemäß bevorzugt. Dieser kann zusätzlich erhöht werden, indem bei der Ölgewinnung auch Blätter des Olivenbaums mitverarbeitet werden, deren Inhaltsstoffe u. a. gegenüber einer Reihe unterschiedlicher Mikroorganismen, darunter Staphylococcus aureus, Streptococcus mutans, Escherichia coli, Candida albicans und Aspergillus niger wirksam sind.

Vorzugsweise werden als aroma- oder inhaltsstoffgebenden Pflanzen, auch in Kombination, verwendet:
Aloe Vera, Ananas, Apfel grün, Avocado, Banane, Basilikum, Bergamotte, Clementine, geröstete Mandeln, getrocknete Peperoncini, getrocknete Tomate, Granatapfel, Grapefruit rot, Grüner Tee, Ingwer, Rosmarin, Kaffee Arabica geröstet, Kakao geröstet, Kakao natur, Karotte, Kiwi, Kokos, Limette, Mais, Mandarine, Melone, Natur Mandeln, Olivenblätter, Orange, Papaja, Paprika grün, Paprika rot, Passionsfrucht, Peperoncini, Pfefferminztee, Rosinen, Sonnenblumenkerne, Tomate, Traube weiß, Zitrone

Diese Aufzählung ist nicht abschließend und soll den Gegenstand des Patentes nicht limitieren.

Das angereicherte Öl kann in unterschiedliche Darreichungsformen, d.h. flüssig, pastös, fest vorliegen. Dies kann durch übliche natürliche Verdickungsmittel erfolgen. Auch können manche Inhaltsstoffe eine Verdickung des Öls herbeiführen, wie z. B. bei der Verwendung von Kokosnüssen.

Die nach dem erfindungsgemäßen Verfahren erhaltenen, angereicherten Öle können in vielfältiger Weise eingesetzt werden, die erst durch die erfindungsgemäße Anreicherung ermöglicht werden, wobei auch industrielle Anwendungen möglich sind.

Das angereicherte Öl lässt sich, je nach zugegebener Pflanze bzw. Frucht, auf vielfältige Weise im Lebensmittelbereich einsetzen, beispielsweise zum Kochen, Braten, als "Dressing", als Würzmittel, als Zutat in Süßspeisen und Getränken.

Nach einer besonders bevorzugten Ausführungsform wird das erfindungsgemäße Öl mit Essig, vorzugsweise einem Kräuteressig, Gewürzessig, einem Fruchtessig oder einer entsprechenden Essigzubereitung mit vorzugsweise auf 2-4 %, besonders bevorzugt mit auf 2,5 - 3,5 % reduzierten Säuregehalt, versetzt, wobei die Essigzubereitung noch mit einem Fruchtdicksaft zur Verstärkung der Geschmacksnote versehen wird. Es können hierbei auch Emulgatoren zur dauerhaften Stabilisierung eingesetzt werden.

Eine weitere besonders bevorzugte Ausführungsform des Öls wird durch Anreicherung von Honig mit dem erfindungsgemäßen Öl erreicht, wobei das Öl bis zur Sättigung des Honigs, je nach Anwendung, zugegeben werden kann. Vorzugsweise ist das Öl dazu mit den Inhaltsstoffen frischer Pflanzen oder Früchte angereichert worden. Bei der Verwendung von getrockneten Pflanzenteilen lässt sich nur schwierig ein stabiles Produkt erhalten.

Die Verwendung eines derartigen Honigs ist äußerst vielfältig. So kann dieser zum Süßen und gleichzeitigen Aromatisieren von Getränken, beispielsweise Tee, von Gebäck oder dergleichen verwendet werden , oder der Honig kann als Lippen- oder Hautpflege, Waschlotion und Badezusatz benutzt werden.

Die vielseitige Einsetzbarkeit des Öls, auch in Süßspeisen, wird, insbesondere bei der Verwendung von Oliven zur Herstellung des Öls dadurch ermöglicht, dass dieses, in erfindungsgemäßer Weise verarbeitet, einen sehr geringen Eigengeschmack hat, d. h. nicht den typischen Olivengeschmack besitzt.

Mit dem erfindungsgemäßen Öl können Fertiggerichte nachträglich aufgewertet und auch noch zusätzlich mit individuellen Geschmacksnoten verfeinert werden. So kann eine Tiefkühlpizza mit gekochtem Schinken mit Ananasaroma zu einer Pizza Hawaii gestaltet werden. Vanilleeis kann je nach Geschmack zu Bananen-, Mandarinen-, Limetten, Pfefferminz- oder Espresso-Eis abgewandelt werden.

Neben der Veränderung oder Intensivierung des Geschmacks bietet das angereicherte Öl Aroma- und Inhaltsstoffe der Früchte oder Kräuter in konzentrierter Form und nebenbei noch die wertvollen Inhaltsstoffe und Fettsäuren der Oliven.

Die Aufwertung von Lebensmitteln ist nicht nur für den privaten Bereich geeignet, sondern lässt sich auch in der Gastronomie sinnvoll einsetzen. In der Gastronomie werden in großem Maße Convenience-Produkte eingesetzt; die, da sie nicht mehr absolut frisch sind, an Aroma verloren haben. Dieser Mangel kann durch die Verwendung der erfindungsgemäßen Öle behoben werden, die beispielsweise aufgesprüht oder aufgetröpfelt werden. Natürlich kann auch der Restaurantbesucher nach Belieben das Öl zum Würzen verwenden.

Im Kosmetikbereich lassen sich die erfindungsgemäß hergestellten Öle sinnvoll einsetzen. So können Hautpflegeprodukte mit wirksamen Inhaltsstoffen aus Pflanzen, wie beispielsweise Aloe Vera, versehen werden und zudem ist der Zusatz von Konservierungsstoffen u. dgl. überflüssig.

Kosmetikprodukte des Öls in Verbindung mit Honig wurden bereits voranstehend beschrieben.

Damit können die Kosmetika besonders umweltschonend hergestellt werden, wobei aufgrund der natürlichen Inhaltsstoffe auch Tierversuche entbehrlich und allergische Reaktionen nicht zu erwarten sind.

Allenfalls Zusatzstoffe wie (Quell)wasser, Bienenwachs, auch andere natürliche Wachse wie Carnauba-Wachs sind verwendbar, und ggf. ätherische Öle können noch zugesetzt werden. Besonders vorteilhaft bei dieser Anwendung ist insbesondere auch die ohnehin schon sehr gute Pflegewirkung des Olivenöts.

Um der Haut Feuchtigkeit zuzuführen, wird das Öl mit Quellwasser versetzt und beispielsweise als Spray verwendet. Dabei können auch Emulgatoren verwendet werden oder das Produkt wird vor der Anwendung geschüttelt. Nach einer besonders bevorzugten Ausführungsform werden dabei 25 ml Öl auf 60 ml Quellwasser verwendet.

Durch die Verwendung von Bienenwachs lassen sich Lippenstifte oder andere geleeartige oder pastöse Hautpflegeprodukte herstellen. Vorzugsweise werden 1-10 Gew.% Bienenwachs in das Öl gegeben.

Bevorzugte Ausführungsformen des erfindungsgemäßen Öls, d. h. des nach dem vorbeschriebenen Verfahren hergestellten Öls, sind wie folgt:
Öl, das mit Essig oder einer Essigzubereitung versetzt ist, wobei der Essig oder die Essigzubereitung vorzugsweise ein Fruchtessig oder eine Fruchtessigzubereitung ist.
Öl, das mit einem Fruchtsaft, vorzugsweise einem Dicksaft versetzt ist.
Öl, das mit Honig vermischt ist, wobei im Honig Öl maximal bis zur Sättigung enthalten ist.
Öl, das mit Quellwasser versetzt ist und ätherische Öle und/oder Emulgatoren enthalten kann.
Öl, das mit natürlichen Wachsen verdickt ist und ätherische Öle enthalten kann.

Die angereicherten Öle können allein oder in Verbindung mit anderen Stoffen als pharmazeutisch wirksame Präparate bzw. Nahrungsergänzungsmittel verwendet werden. Die Wirksamkeit von insbesondere Olivenöl zur Vorbeugung gegen diverse Krebsarten, wie Brust-, Lungen- und Prostatakrebs, zur Regulierung des Cholesterinspiegels und zur Vorbeugung kardio-vaskulärer Erkrankungen lässt sich durch die Auswahl entsprechender Pflanzen, mit deren Inhaltsstoffen die Öle angereichert werden, steigern. Beispielsweise kann durch die Anreicherung mit den Inhaltsstoffen aus der Tomate die Wirksamkeit auf das kardio-vaskuläre System nochmals vergrößert werden.

Das angereicherte Öl ist aufgrund des hohen Antioxidantiengehalts und des Gehalts an antimikrobiellen Wirkstoffen sehr gut als Konservierungsmittel für die verschiedensten Bereiche geeignet. So sind die angereicherten Öle als Konservierungsstoffe für Lebensmittel, Kosmetika und Arzneimittel geeignet. Insbesondere Lebensmittel, die üblicherweise sehr schnell verarbeitet werden müssen, wie frischer Fisch oder Hackfleisch, können mit dem erfindungsgemäßen Öl über mehrere Tage frisch gehalten werden.

Weitere vorteilhafte Ausgestaltungen der Erfindung sind in den Unteransprüchen gekennzeichnet.

## Patentansprüche

1. Verfahren zur Übertragung und Konservierung von Aroma- und Inhaltsstoffen von frischen, getrockneten und/oder verarbeiteten Früchten, Samen, Pflanzen und/oder Pflanzenteilen auf ein Öl,
**dadurch gekennzeichnet,**
**dass** zumindest bei einem Verfahrensschritt während der Herstellung des Öls aus ölhaltigen Früchten, Samen, Pflanzen oder Pflanzenteilen, die aroma- und Inhaltsstoffgebenden Früchte, Samen, Pflanzen, Pflanzenteile, verarbeitete Pflanzen und/oder Pflanzenteile hinzugefügt und mit verarbeitet werden.

2. Verfahren nach Anspruch 1, **dadurch gekennzeichnet, dass** die zur Herstellung des Öls dienenden ölhaltigen Früchte, Samen, Pflanzen oder Pflanzenteile
a) mechanisch zu einem Brei zerkleinert werden,
b) der Brei gerührt wird,
c) das im Brei enthaltene Öl-/Wassergemisch vom Brei abgetrennt wird,
wobei die aroma- und Inhaltsstoffgebenden Früchten, Samen, Pflanzen, Pflanzenteile, verarbeitete Pflanzen und/oder Pflanzenteile in Schritt a) und/oder b) zugegeben und mitverarbeitet werden und/oder nach Durchführung des Schrittes c) dem Öl-/Wassergemisch zugefügt werden.

3. Verfahren nach Anspruch 1 oder 2, **dadurch gekennzeichnet, dass** das nach dem Abtrennen der festen Bestandteile bzw. des Breis erhaltene Öl-/Wassergemisch aufgetrennt wird.

4. Verfahren nach Anspruch 2, **dadurch gekennzeichnet, dass** das in Schritt c) erhaltene Öl-/Wassergemisch mit den aroma- und Inhaltsstoffgebenden Früchten, Samen, Pflanzen, Pflanzenteile, verarbeitete Pflanzen und/oder Pflanzenteile für eine vorgegebene Zeitdauer in Kontakt bleibt und währenddessen stetig durchmischt werden kann.

5. Verfahren nach einem der Ansprüche 2 bis 4, **dadurch gekennzeichnet, dass** in Schritt c) das Öl-/wassergemisch durch Dekantieren gewonnen wird und das erhaltene Öl oder Öl-/Wassergemisch zentrifugiert sowie gegebenenfalls filtriert wird.

6. Verfahren nach einem der Ansprüche 1 bis 5, **dadurch gekennzeichnet, dass** das Verfahren bei Temperaturen unterhalb von 28 °C, vorzugsweise unterhalb 25 °C durchgeführt wird.

7. Verfahren nach einem der Ansprüche 1 bis 6, **dadurch gekennzeichnet, dass** als ölhaltige Frucht Oliven oder Traubenkerne eingesetzt werden.

8. Verfahren nach Anspruch 7, **dadurch gekennzeichnet, dass** Oliven der Sorte Coratina verwendet werden.

9. Verfahren nach Anspruch 7 oder 8, **dadurch gekennzeichnet, dass** die Oliven in grünem, unreifem Zustand verwendet werden.

10. Verfahren nach einem der Ansprüche 1 bis 9,**dadurch gekennzeichnet, dass** die aroma- oder Inhaltsstoffgebenden Pflanzen aus folgender Gruppe ausgewählt sind:
Blätter des Olivenbaum, Aloe Vera, Ananas, Apfel grün, Avocado, Banane, Basilikum, Bergamotte, Clementine, geröstete Mandeln, getrocknete Peperoncini, getrocknete Tomate, Granatapfel, Grapefruit rot, Grüner Tee, Ingwer, Rosmarin, Kaffee Arabica geröstet, Kakao geröstet, Kakao natur, Karotte, Kiwi, Kokos, Limette, Mais, Mandarine, Melone, Natur Mandeln, Olivenblätter, Orange, Papaja, Paprika grün, Paprika rot, Passionsfrucht, Peperoncini, Pfefferminz, Rosinen, Sonnenblumenkerne, Tomate, Traube weiß, Zitrone.

11. Öl hergestellt nach dem Verfahren gemäß einem der Ansprüche 1 bis 10.

12. Verwendung des angereicherten Öls gemäß Anspruch 11 in Kosmetika oder als Kosmetikum.

13. Verwendung des angereicherten Öls gemäß Anspruch 11 als Konservierungsmittel.

14. Verwendung des angereicherten Öls gemäß Anspruch 11 zur Herstellung eines pharmazeutischen Präparates.

15. Verwendung des angereicherten Öls gemäß Anspruch 11 als Nahrungsergänzungsmittel.

## Claims

1. A method for transferring and preserving aromatic substances and constituents of fresh, dried and/or processed fruits, seeds, plants and/or plant parts to an oil and thus to provide an oil **characterized in that** at least in one process step during the production of oil from oil-bearing fruits, seeds, plants or plant parts, the fruits, seeds, plants, plant parts, processed plants and/or plant parts which provide the aromatic substances and constituents are added and also processed.

2. The method according to claim 1, **characterized in that** the oil-containing fruits, seeds, plants or plant parts used to produce the oil
a) are mechanically comminuted to form a puree,
b) the puree is stirred,
c) the oil/water mixture contained in the puree is separated from the puree,
wherein the the fruits, seeds, plants, plant parts, processed plants and/or plant parts which provide the aromatic substances and constituents are added and also processed in step a) and/or b) and/or after carrying out step c), are added to the oil/water mixture.

3. The method according to claim 1 or 2, **characterized in that** the oil/water mixture obtained after separating the solid constituents or the puree is separated.

4. The method according to claim 2, **characterized in that** the oil/water mixture obtained in step c) remains in contact with the fruits, seeds, plants, plant parts, processed plants and/or plant parts which provide the aromatic substances and constituents for a predefined time and during this time can be continuously thoroughly mixed.

5. The method according to any one of claims 2 to 4, **characterized in that** in step c) the oil/water mixture is obtained by decanting and the oil or oil/water mixture obtained is centrifuged and optionally filtered.

6. The method according to any one of claims 1 to 5, **characterized in that** the process is carried out at a temperature below 28°C, preferably below 25°C.

7. The method according to any ane of claims 1 to 6, **characterized in that** olives or grape seeds are used as the oil-containing fruit.

8. The method according to claim 7, **characterized in that** the olives of the species Coratina are used.

9. The method according to claim 7 or 8, **characterized in that** the olives are used in the green, unripe state.

10. The method according to any one of claims 1 to 9, **characterized in that** the plants which provide the aromatic substances and constituents are selected from the following group:
leaves of the olive tree, aloe vera, pineapple, green apple, avocado, banana, basil, bergamot, clementine, roasted almonds, dried peperoncini, dried tomato, pomegranate, red grapefruit, green tea, ginger, rosemary, roasted Arabica coffee, roasted cocoa, natural cocoa, carrot, kiwi, coconut, lime, maize, mandarin, melon, natural almonds, olive leaves, orange, papaya, green paprika, red paprika, passion fruit, peperoncini, peppermint, raisins, sunflower seeds, tomato, white grape, lemon.

11. Oil prepared by the method according to one of claims 1 to 10.

12. Use of the enriched oil according to claim 11 in cosmetics or as a cosmetic.

13. Use of the enriched oil according to claim 11 as preservative.

14. Use of the enriched oil according to claim 11 to produce a pharmaceutical preparation.

15. Use of the enriched oil according to claim 11 as a food supplement.

## Revendications

1. Procédé de transfert et de conservation d'arômes et d'ingrédients de fruits, graines, plantes et/ou parties de plantes frais, séchés et/ou transformés pour obtenir une huile,
**caractérisé en ce que**, au moins lors d'une étape opératoire pendant la fabrication de l'huile à partir de fruits, graines, plantes ou parties de plantes contenant de l'huile, les fruits, graines, plantes et/ou parties de plantes donnant les arômes et ingrédients sont ajoutés et transformés en même temps.

2. Procédé selon la revendication 1, **caractérisé en ce que** les fruits, graines, plantes ou parties de plantes servant à la fabrication de l'huile
a) sont broyés mécaniquement en bouillie,
b) la bouillie est remuée,
c) le mélange d'huile et d'eau contenu dans la bouillie est isolé de la bouillie,
les fruits, graines, plantes et/ou parties de plantes transformés donnant les arômes et ingrédients sont ajoutés et transformés en même temps dans l'étape a) et/ou b) et/ou incorporés après réalisation de l'étape c) dans le mélange d'huile et d'eau.

3. Procédé selon la revendication 1 ou 2, **caractérisé en ce que** le mélange d'huile et d'eau obtenu après l'isolement des composantes solides ou de la bouillie est isolé.

4. Procédé selon la revendication 2, **caractérisé en ce que** le mélange d'huile et d'eau obtenu dans l'étape c) reste en contact avec les fruits, graines, plantes, parties de plantes transformés donnant des arômes et ingrédients pendant une durée déterminée et peut être mélangé constamment pendant ce temps.

5. Procédé selon une des revendications 2 à 4, **caractérisé en ce que** le mélange d'huile et d'eau est produit par décantation dans l'étape c) et que le mélange d'huile et d'eau obtenu est centrifugé et filtré le cas échéant.

6. Procédé selon une des revendications 1 à 5, **caractérisé en ce que** le procédé est réalisé à des températures inférieures à 28° C, de préférence inférieures à 25° C.

7. Procédé selon une des revendications 1 à 6, **caractérisé en ce qu'**on utilise comme fruits contenant de l'huile des olives ou des pépins de raisin.

8. Procédé selon la revendication 7, **caractérisé en ce qu'**on utilise des olives de variété Coratina.

9. Procédé selon la revendication 7 ou 8, **caractérisé en ce que** les olives sont utilisées à l'état vert et non mûr.

10. Procédé selon une des revendications 1 à 9, **caractérisé en ce que** les plantes donnant des arômes et ingrédients sont sélectionnées parmi le groupe suivant :
feuilles d'olivier, aloe vera, ananas, pomme verte, avocat, banane, basilic, bergamote, clémentine, amandes grillées, piments cerises séchés, tomates séchées, grenade, pamplemousse rouge, thé vert, gingembre, romarin, café arabica torréfié, cacao torréfié, cacao nature, carotte, kiwi, noix de coco, citron vert, maïs, mandarine, melon, amandes nature, feuilles d'olivier, orange, papaye, poivron vert, poivron rouge, fruit de la passion, piment cerise, menthe poivrée, raisins secs, graines de tournesol, tomate, raisin blanc, citron.

11. Huile fabriquée selon le procédé selon une des revendications 1 à 10.

12. Utilisation de l'huile enrichie selon la revendication 11 dans des cosmétiques ou comme cosmétique.

13. Utilisation de l'huile enrichie selon la revendication 11 comme conservateur.

14. Utilisation de l'huile enrichie selon la revendication 11 pour produire une préparation pharmaceutique.

15. Utilisation de l'huile enrichie selon la revendication 11 comme complément alimentaire.
